Europäisches Patentamt

European Patent Office

Office européen des brevets

(19)

(11) Publication number: **0 113 074**
**B1**

(12) **EUROPEAN PATENT SPECIFICATION**

(45) Date of publication of patent specification: **25.05.88**

(51) Int. Cl.⁴: **H 01 L 27/14, H 01 L 31/10, H 03 K 19/14**

(21) Application number: **83112244.5**

(22) Date of filing: **06.12.83**

(54) **Method of logically combining optical signals.**

(30) Priority: **30.12.82 US 454747**

(43) Date of publication of application:
**11.07.84 Bulletin 84/28**

(45) Publication of the grant of the patent:
**25.05.88 Bulletin 88/21**

(84) Designated Contracting States:
**DE FR GB**

(56) References cited:
**US-A-3 040 178**
**US-A-3 402 300**
**US-A-3 519 844**
**US-A-4 127 862**
**ELECTTRONICS, 8th September 1981, pages 42-44, New York, US; "Optical AND gate has current output"**
**INTERNATIONAL ELECTRON DEVICES MEETING TECHNICAL DIGEST, 13th-15th December 1982, San Francisco, pages 334-337, IEEE, New York, US; F. CAPASSO et al.;**

(73) Proprietor: **International Business Machines Corporation**
**Old Orchard Road**
**Armonk, N.Y. 10504 (US)**

(72) Inventor: **Chappell, Terry Ivan**
**RDF Adele Court**
**Amawalk New York 10501 (US)**
Inventor: **Woodall, Jerry MacPherson**
**336 Cherry Street**
**Bedford Hills New York 10507 (US)**

(74) Representative: **Hobbs, Francis John**
**IBM United Kingdom Limited**
**Intellectual Property Department**
**Hursley Park Winchester**
**Hampshire SO21 2JN (GB)**

(56) References cited:
**"Superlattice, graded band gap, channeling and staircase avalanche photodiodes towards a solid-state photomultiplier"**
**ELECTRONICS LETTERS, vol. 16, no. 22, 23rd October 1980, pages 836-837, Hitchins, GB; R.J. MALIK et al.: "Planar-doped barriers in GaAs by molecular beam epitaxy"**

Courier Press, Leamington Spa, England.

## Description

This invention relates to a method of logically combining optical signals.

As speed and device densities increase, it is becoming more advantageous to transmit information signals in light beams and to route those light beams in conduits such as optical fibres. Logic signals that are in optical form are then logically combined in a device that produces an electrical signal. The logical combination operation, however, frequently introduces added structure and delays incompatible with speed capability requirements.

Logic signals in optical form have been combined in a single semiconductor structure wherein each logic variable is of an individual wavelength. Such a structure is illustrated in Electronics, September 8, 1981, page 42. In this complex semiconductor heterostructure, diffusion is the carrier transport mechanism so that there will be a limit on speed of responsiveness.

In a further application filed concurrently herewith and not published prior to the present application, a photodiode is described which comprises an undoped semiconductor layer having a thickness of the order for ballistic transport bounded by a tunnelling barrier that is of the order of the mean free path of a carrier in the tunnelling portion of the semiconductor material. In this device the carrier transport mechanism is drift and a much higher output voltage and higher speed of response is achieved than in devices in which the carrier transport mechanism is diffusion.

The invention provides a method of logically combining optical signals of individual wavelengths comprising directing the signals simultaneously at an incident top surface of a semiconductor photodetector comprising an integrated series of photodiodes each consisting of an undoped light-absorbing semiconductor region contiguous with one component region of a pair of doped semiconductor regions forming a quantum mechanical tunnelling pn junction, the combined thickness of the pair of doped semiconductor regions being of the order of the mean free path length of a charge carrier therein and the undoped light-absorbing region of a succeeding diode in the series being contiguous with the other component region of the pair of doped semiconductor regions of the preceding diode in the series, and the light-absorbing regions of each different one of the photodiodes being such as to absorb a high proportion of an individual one of the individual wavelengths and a low proportion of the other individual wavelengths. Particular embodiments of the invention are set out in the dependent claims.

How the invention can be carried out will now be described by way of example, with reference to the accompanying drawings, in which:—

Fig. 1 represents a semiconductor structure suitable for performing a logical AND operation on two optical signals of different wavelengths; and

Fig. 2 is an energy diagram correlated with Fig. 1, on which absorption of light of different wavelengths is indicated.

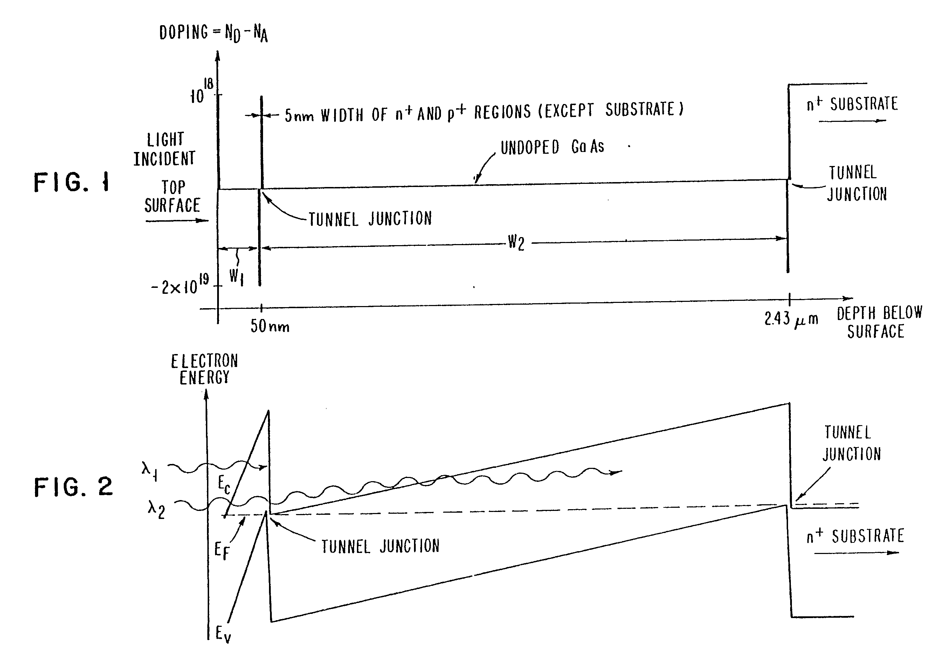

A two logic variable-coincident or "AND" structure (Fig. 1) has an individual planar undoped semiconductor region for each logic variable arranged serially under a light incident top surface. The logic variables are introduced through the light incident surface as an individual wavelength signal for each variable. The individual wavelength logic variable signals may be delivered independently or modulated in a single beam. The logic variable signals enter from the light incident surface along the X or ordinate axis. In Fig. 1, the doping of the structure is indicated along the Y or abscissa axis. The degenerate doping of the tunnelling boundaries occurs over such a short distance that a line is shown. The logic variable conversion undoped region adjacent the light incident surface would be tailored for the shorter wavelength and the region further below the light incident surface for the longer wavelength.

The logic variable conversion undoped regions are bounded by quantum mechanical tunnelling junctions having a thickness of the order of the mean free path of a carrier in the tunnelling junction region. In most materials and particularly in GaAs, 5 nm (50 Å) is a sufficient distance. In Fig. 1, the boundary tunnelling junctions are shown as 50 nm (50 Å) thick regions of $n^+$ and $p^+$ adjacent each other. The combined $n^+$ and $p^+$ distances of 10 nm (100 Å) are in toto of the order of the mean free path of a carrier in the tunnelling region of the GaAs material.

The thickness of each logic variable conversion region is tailored to absorb of the order of 90% of the light of the wavelength of the logical operator to which it is to be responsive.

The total depth from the light incident surface to the substrate is at least the depth of penetration of the light wavelength for the logic variable to be absorbed in the region next to the substrate. This is the longest wavelength logic variable signal.

The dimensions and the wavelengths are selected to discriminate between logical variables such that of the order of 90% of all the light of one wavelength is absorbed yet will not produce more than of the order of 10% of the current in the region for another wavelength. The 90% and 10% values are selected for good signal to noise ratios. In conditions where needed closer discrimination can be achieved with tighter structure and wavelength limitations.

Referring to Figs. 1 and 2 together, the first active region is arranged to produce a large photocurrent response to light at wavelength $\lambda_1$ yet only has less than 10% of the photocurrent produced by light at wavelength $\lambda_2$ which passes through the region for $\lambda_1$. Similarly, the region further from the surface responds to light at wavelength $\lambda_2$. $\lambda_1$ is less than $\lambda_2$. Tailoring of the photocurrent response of the two active regions is done by varying either singly or together the thickness or band gap through material composition of each region. The greater the separation in wavelengths between the signals representing logic variables the less strenuous the discriminating ability between the regions need be. Where the logic variable requirements are such that the wavelengths become closer, both band gap and thickness differentiation become more essential.

The logical variable $\lambda$ selection and the conversion region parameters should be such that each separate region has a quantum efficiency for the desired wavelength that is such that the effects of other wavelengths do not exceed 10%. For the device embodiment of Fig. 1 using single undoped GaAs material, the quantum efficiency of the first logic variable conversion region having a thickness $W_1$ is 0.5 μm for a $\lambda_1$ of 0.425 μm is 90% and for a $\lambda_2$ of 0.78 μm the efficiency would be 6.9% in the same region. In the second logic variable conversion region having a thickness $W_2$ of 1.9 μm, the quantum efficiency would be 90% for $\lambda_2$ but would be 10% for $\lambda_1$ in that region.

For undoped GaAs material where the doping level is less than $10^{16}$ atoms per $cm^3$, electric fields of 3500 V/cm may exist in each undoped region. The thickness of each region is selected so that high absorption and high photocurrent for the particular wavelength occurs. Since any other logic variable representing wavelengths would provide only 10% of the photocurrent, there would be output signal level photocurrent through the combined regions only when the wavelength signal for each region in combination is present.

In operation, under no bias voltage, the "AND" logic operator is developed between an ohmic contact to the substrate and a surface contact such as a grid or transparent ohmic contact. When the device is illuminated by two distinct monochromatic light wavelengths, which may be contained in a single beam, each representing a logical variable appreciable output signal level photocurrent can flow through the device. If only one of either of the wavelengths is present, very little photocurrent can flow since the regions are in series. Thus, the device acts as an optical AND gate, providing an appreciable current only when both light wavelengths are present. In extending the principle set forth it will be apparent that any number of logic variable conversion regions in series will result in an output logic operator current being the logical coincidence or "AND" function of all the input logic variable wavelengths.

The structure of the converter provides a high field as a result of the undoped regions being bounded by tunnel junctions. The high field in turn accelerates carriers produced by the specific logic variable light wavelength. The undoped condition of the region also minimizes carrier scattering as the light produced carriers move toward the boundary. The undoped region is thick enough to provide maximum carriers produced by the light wavelength for that region.

These conditions permit drift rather than diffusion to be the dominant carrier transport mechanism so that logic signal results with only drift limited carrier transport delay are achieved. The tunnelling boundaries provide low impedance to the drift transport of the carriers and are thin enough that no significant delay is introduced.

Since the photogenerated carriers are transported by drift rather than diffusion, a very fast transient response is achieved so that extra logic delay does not occur.

Referring again to Figs. 1 and 2, the limiting transient response of the device is governed by the transit time of the thickest undoped region which has the thickness $W_2$. This is because the photocarriers are collected simultaneously by both the regions so that with substantially equal crystal conditions the thickest region will be the slowest. For the device of Fig. 1, the transit time follows the relationship in Equation 1 using the values for GaAs and the dimensions of Fig. 1.

Eq. 1

$$t_r = \frac{W_2}{V_s} = \frac{24 \times 10^{-5} \text{ cm}}{10^7 \text{ cm/sec}} = 24 \text{ picoseconds}$$

where
$t_r$ is the transit time, and
$v_s$ is the saturation drift velocity.

In the light of the principles of physics employed in the structure that have been set forth, it will be apparent that many substitutions will be available. For example, very thin regions could be achieved by growing epitaxially the two regions from two different semiconductor materials which are selected to have high absorption coefficients at two different wavelengths. In other words, if the undoped GaAs region material with the thickness $W_1$ is replaced by a material such as GaAlAs with a larger band gap, the other region is made of GaAs can have a smaller thickness $W_2$. This would permit more regions within the depth of penetration capability of certain frequencies.

The device of Fig. 1 can be made by the technique of molecular beam epitaxy to grow the undoped regions as a single crystal. The tunnelling barriers can be formed between regions of the crystal using the planar doping molecular beam epitaxial techniques such as is described in Electronics Letters 16, 22, page 836, October 23, 1980.

The $p^+$ regions of the device are preferably more safely made using Mg as the dopant rather than Be which is standard in the art but which being poisonous is a source of handling difficulties. Efficient doping with Mg may be achieved using a volatile oxide suppression technique as described in the article entitled "Volatile Metal Oxide Incorporation in layers of GaAs, $Ga_{1-x} Al_x$ and related Compounds grown by Molecular Beam Epitaxy" published in pages 427 to 429 of Applied Physics Letters, 15 March 1981.

In order to facilitate explanation, the doping profile and materials composition have been set forth for a coincidence or "AND" logic function in the material GaAs which is chosen for its high optical-to-electrical conversion efficiency and mobility. It will, however, be clear to one skilled in the art in the light of the principles set forth that other semiconductor materials may be employed. Similarly, since all logic operators can be achieved by combinations of coincidence or "AND", presence or "OR" and denial or "NOT", and since NOT can be achieved by a presence signal interrupting an existing bias and since presence can be achieved by a single photodetector; the achieving of coincidence or "AND" being the most complex one skilled in the art can readily translate the principles set forth to logic involving combinations of "AND", "OR" and "NOT".

**Claims**

1. Method of logically combining optical signals of individual wavelengths comprising directing the signals simultaneously at an incident top surface of a semiconductor photodetector comprising an integrated series of photodiodes each consisting of an undoped light-absorbing semiconductor region contiguous with one component region of a pair of doped semiconductor regions forming a quantum mechanical tunnelling pn junction, the combined thickness of the pair of doped semiconductor regions being of the order of the mean free path length of a charge carrier therein and the undoped light-absorbing region of a succeeding diode in the series being contiguous with the other component region of the pair of doped semiconductor regions of the preceding diode in the series, and the light-absorbing regions of each different one of the photodiodes being such as to absorb a high proportion of an individual one of the individual wavelengths and a low proportion of the other individual wavelengths.

2. A method as claimed in claim 1, in which each different light-absorbing region is such as to absorb approximately 90% of one of the individual wavelengths and no more than approximately 10% of each of the other individual wavelengths.

3. A method as claimed in claim 1 or claim 2, in which the optical signals have 0.425 µm and 0.78 µm wavelengths respectively and in which the photodetector comprises a series of two photodiodes integrated in a body of GaAs, the undoped light-absorbing region adjacent the incident surface being 0.5 µm thick and the undoped light-absorbing region remote from the incident surface being 1.9 µm thick.

**Patentansprüche**

1. Verfahren zur logischen Kombination optischer Signale unterschiedlicher Wellenlänge, dadurch gekennzeichnet, daß die Signale gleichzeitig auf eine Lichteinfalls-Oberfläche eines Halbleiter-Photodetektors mit einer integrierten Photodiodenreihe geleitet werden, welche jeweils aus einem nichtdotierten, lichtabsorbierenden Halbleiterbereich bestehen, der an einen aus zwei dotierten Halbleiterbereichen zusammengesetzten Bereich angrenzt, so daß ein quantenmechanischer PN-Tunnelübergang gebildet wird, wobei die kombinierte Dicke der beiden dotierten Halbleiterbereiche innerhalb der mittleren freien Weglänge eines darin befindlichen Ladungsträgers leigt und der nichtdotierte, lichtabsorbierende Bereich einer in der Reihe nachfolgenden Diode an den anderen aus den beiden dotierten Halbleiterbereichen zusammengesetzten Bereich der in der Reihe vorhergegangenen Diode angrenzt, und wobei die lichtabsorbierenden Bereiche jeder unterschiedlichen Photodiode einen hohen Anteil einer der einzelnen Wellenlängen und einen geringen Anteil der anderen einzelnen Wellenlängen absorbiert.

2. Verfahren nach Anspruch 1, dadurch gekennzeichnet, daß jeder unterschiedliche, lichtabsorbierende Bereich ca. 90% einer der einzelnen Wellenlängen und nicht mehr als 10% jeder der übrigen einzelnen Wellenlängen absorbiert.

3. Verfahren nach Anspruch 1 oder 2, dadurch gekennzeichnet, daß die optischen Signal jeweils 0,425 µm und 0,78 µm Wellenlänge aufweisen und daß der Photodetektor eine Reihe von zwei Photodioden aufweist, welche in einen GaAs-Körper integriert sind, wobei der neben der Einfallsfläche befindliche nichtdotierte, lichtabsorbierende Bereich 0,5 µm dick ist und der von der Einfallsfläche entfernt liegende nichtdotierte, lichtabsorbierende Bereich 1,9 µm dick ist.

**Revendications**

1. Procédé pour combiner de façon logique des signaux optiques de longueurs d'onde individuelles comportant la direction simultanée des signaux sur une surface supérieure incidente d'un photodétecteur à semiconducteurs comportant une série intégrée de photodiodes constituées chacun par une région semiconductrice non dopée d'absorption de lumière contiguë à une région composante d'une paire de régions semiconductrices dopées formant une jonction pn à effet tunnel selon la mécanique quantique, l'épaisseur combinée de la paire de régions semiconductrices dopées étant de l'ordre de la longueur moyenne du chemin libre d'un porteur de charge à l'intérieur de celle-ci et la région non dopée d'absorption de lumière d'une diode suivante dans la série étant contiguü à l'autre région composante de la paire de régions semiconductrices dopées de la diode précédente dans la série, et les régions d'absorption de lumière de chaque différente photodiode desdites photodiodes étant telles qu'elles absorbent une forte proportion de l'une des longueurs d'onde individuelles et une faible proportion des autres longueurs d'ondes individuelles.

2. Procédé selon la revendication 1, dans lequel chaque région différente d'absorption de lumière est telle qu'elle absorbe approximativement 90% de l'une des longueurs d'onde individuelles et pas plus de 10% environ de chacune des autres longueurs d'onde individuelles.

3. Procédé selon la revendication 1 ou la revendication 2, dans lequel les signaux optiques ont des longueurs d'onde de 0,425 µm et de 0,78 µm respectivement, et dans lequel le photodétecteur comporte une série de deux photodiodes intégrées dans un corps de GaAs, la région non dopée absorbant la lumière, adjacente à la surface incidente, ayant une épaisseur de 0,5 µm et la région non dopée absorbant la lumière éloignée de la surface incidente, ayant une épaisseur de 1,9 µm.

FIG. 1

FIG. 2